# EUROPEAN PATENT SPECIFICATION

(11) **EP 0 323 708 B1**
(45) Date of publication and mention of the grant of the patent: **11.10.1995**
(21) Application number: 88311331.8
(22) Date of filing: 30.11.1988
(51) Int. Cl.: A61K 39/15

(54) **Rotavirus reassortant vaccine**
Reassortantes Rotavirusvakzin
Vaccin à base de réassortants de Rotavirus

(30) Priority: 30.11.1987 US 126477
(43) Date of publication of application: 12.07.1989
(73) Proprietor: THE WISTAR INSTITUTE OF ANATOMY AND BIOLOGY, Philadelphia, PA 19104 (US); THE CHILDREN'S HOSPITAL OF PHILADELPHIA, Philadelphia, Pennsylvania 19104 (US)
(72) Inventor: Plotkin, Stanley A., Pennwynne, PA 19151 (US); Clark, H. Fred, Wynnewood, PA 19096 (US); Offit, Paul, Philadelphia, PA 19146 (US)
(74) Representative: Hale, Stephen Geoffrey

(56) References cited:
- EP-A- 0 130 906
- EP-A- 0 192 404
- JOURNAL OF VIROLOGY, vol. 53, no. 3, March 1985, American Society for Microbiology, US; K. MIDTHUN et al., pp. 949-954#
- VIROLOGY, vol. 112, 1981, Academic Press Inc., US; A.R. KALICA, pp. 385-390#
- JOURNAL OF CLINICAL MICROBIOLOGY, vol. 24, no. 5, November 1986, American Society for Microbiology, US; K. MIDTHUN et al., pp. 822-826#
- JOURNAL OF VIROLOGY, vol. 60, no. 2, November 1986, American Society for Microbiology, US; P.A. OFFIT et al., pp. 491-496#
- PROCEEDINGS OF THE NATIONAL ACADEMY OF SCIENCES USA, vol. 82, December 1985; Y. HOSHINO et al., pp. 8701-8704#
- BIOLOGICAL ABSTRACTS, vol. 84, no. 10, 1987, Biological Abstracts Inc., Philadelphia, PA (US); H.F. CLARK et al., no. 104394#
- JOURNAL GEN. VIROL., vol. 68, 1987, SGM, GB; A. GRAHAM et al., pp. 115-122#
- VIROLOGY, vol. 163, 1988; pp. 26-32#
- IMMUNOLOGICAL INVESTIGATIONS, vol. 18, nos. 1-4, 1989; pp. 571-581#
- JOURNAL OF VIROLOGY, vol. 57, no. 1, 1986; pp. 46-49#

## Description

The present invention refers generally to novel rotavirus reassortants, vaccines employing the novel reassortants and methods for their preparation and administration. More particularly, the invention provides a reassortant in which either the gene encoding the neutralization antigen originally known as v.p.3 but later known as v.p.4 or the gene encoding the v.p.7 neutralization antigen is derived from bovine rotavirus of the WC3 strain, the other of these two genes is derived from a selected human rotavirus, further genes being derived from the bovine strain and/or from the human rotavirus.

### Background of the Invention

Rotaviruses are the single most important etiologic agent of infectious gastroenteritis (diarrhea), which is the leading cause of infant death in the world. Of the estimated 5 to 10 million infant deaths yearly caused by acute infectious gastroenteritis [Walsh et al, New Eng. J. Med., 301:967 (1979)], rotaviruses cause between 10 to 40% of the yearly total [deZoysa and Feachem, Bull WHO, 63:569 (1958)]. Although infant mortality rates in developing countries due to infectious gastroenteritis are staggering, rotaviruses also cause a majority of such cases leading to hospitalization in developed nations, such as the U.S.. Thus, rotavirus-induced infectious gastroenteritis is one of the ten leading causes of infant death, even in developed nations [Ho et al, 27th Interscience Conf. Antimicrobiol Agents Chemotherapy, p2 (1987)].

Serological studies indicate that virtually all infants, worldwide, are infected with rotavirus within the first two or three years of life. While rotavirus-induced disease occurs most often between the ages of 6 and 24 months, occurance of the disease in under-developed countries is common at ages less than 6 months. Rotavirus infection is transmitted from person to person by the fecal-oral route, and typically has a one to three day incubation period. Rotavirus infection exacts a severe toll on infants, while most adult infections are mild or asymptomatic. Additionally rotaviruses have been identified as a cause of neonatal diarrhea in virtually all species of domestic animals. Rotaviruses of primate and bovine origin are spherical viruses, about 70 nm in diameter and characterized by a double capsid structure. The rotavirus genome has eleven segments of doublestranded RNA and an RNA polymerase. Each segment of RNA is a gene that codes for a single protein gene product. A majority of the presently identified animal and human rotaviruses are designated as Group A rotaviruses, and share a common cross-reactive antigen [Estes et al, Immunochemistry of Viruses, Elsevier, Amsterdam:389 (1984)]. Different species of rotaviruses are, however, distinguishable by distinct serotype-specific virus surface antigens. These surface antigens are most easily detected in conventional serum-neutralization (SN) tests. In a serum neutralization test, an antiserum prepared against a purified virus of a specific serotype scores a higher SN titer with a virus of a homologous serotype than with a virus of a heterologous serotype. Among human rotaviruses, at least six serotypes are now recognized--serotypes 1, 2, 3, 4, M69 and a new serotype WI61. [Wyatt et al, Infect and Immun, 37:110 (1983); Matsuno et al, J. Virol., 54:623 (1985) Clark et al, J. Clin Microbiol., 25: 1757 (1987)].

Presently available knowledge on the cross-immunity of various serotypes in animals and humans has provided contradictory results with regard to the necessity for serotype specific antigenic stimulation to provide immmune protection against rotavirus infection. See, for example, several reports on vaccine challenge studies in animals [Wyatt et al, Science, 203:548 (1979); Zissis et al, J. Inf. Dis., 148:1061 (1983); Sheridan et al, J. Inf. Dis., 149:434 (1984)]. See also several reports on rotavirus vaccines evaluated in animals and humans [Mebus et al, J.A.V.M.A., 163:880 (1973); Thurber et al, Canad, Vet. J., 17:197 (1976); Vesikari et al, Lancet, 2:807 (1983); De Mol et al, Lancet, 2:108 (1986); Clark et al, Amer. J. Dis. Children, 140:350 (1986); Kapikian et al, Vaccines, New York, Cold Spring Harbor Lab., 357 (1985); Losonsky et al, Ped Inf. Dis., 5:25 (1986); Santosham et al, 27th Int. Conf. Antimicrobiol. Agents and Chemotherapy, p. 99 (1987)]. These studies have led some authorities to speculate that an ideal rotavirus vaccine may need to include serotype-specific antigens representative of all prevalent human rotavirus serotypes.

Human rotaviruses have not been avidly pursued for vaccine use because cell culture-adapted human rotaviruses replicate inefficiently, particularly in cells considered to be acceptable as human vaccine substrates. Additionally, the potential pathogenicity of human rotavirus isolates is largely unknown. To overcome these disadvantages, genetic recombination of human rotaviruses with animal rotaviruses has been suggested. Such recombination, called "reassortment" in rotaviruses, is possible because of the segmented nature of the RNA genome of rotavirus and its high frequency of gene reassortment during coinfection. For example, when cells in culture are mixedly infected with two different rotavirus strains, spontaneous mixing, or gene reassortment, of gene segments of the two rotaviruses often occurs. Individual progeny viruses derived from such mixed infections are selected by isolating individual plaques generated by plaque assay. The parental origin of each gene segment of the progeny virus can be determined by the characteristic rate of migration during polyacrylamide gel electrophoresis (PAGE) of each gene segment.

Reassortant rotaviruses produced and proposed as human vaccine candidates have included primarily animal origin rotaviruses in which a single gene product, the 38,000 dalton v.p. 7 antigen of gene 9 or 8, has been replaced by the v.p.7 encoding gene of a human serotype rotavirus. The v.p.7 protein functions as a major serotype-specific antigen, operative in serum neutralizing tests. It is capable of inducing serotype-specific neutralizing antibody and inducing serotype-specific immune protection in a mouse system against rotavirus disease. See, e.g., Offit et al, J. Virol., 60:491 (1986)]. Such reassortants containing the v.p.7 encoding gene include reassortants based upon U. K. strain bovine rotavirus with a v.p.7 of serotype 1, 2, 3 or 4; or based upon rhesus rotavirus (RRV) with a v.p.7 of serotype 1, 2, or 4 [Midthun et al, J. Virol., 53:949 (1985); Midthun et al J. Clin. Microbiol., 24:822 (1986) and US-A-4,571,385].

### Summary of the Invention

The present invention provides reassortant rotaviruses useful as vaccines against human rotavirus infection. One class of reassortants according to the invention contains at least the gene (gene segment 4) from the bovine rotavirus strain WC3 (or an isolate thereof) encoding the neutralization antigen originally known as v.p.3 but later as v.p.4 and the gene from a selected human rotavirus encoding the v.p.7 neutralization antigen. Reassortants provided according to this aspect of the invention contain at least one other human rotavirus gene segment, as well as the v.p.7 encoding gene segment, provided that the segment encoding the antigen originally known as v.p.3 but later as v.p.4 is derived from the WC3 bovine strain. One or more other WC3 bovine strain gene segments may also be present.

The reassortants of this class in accordance with the present invention employ the gene segment 4 from the bovine strain WC3 rotavirus or its progeny; as described in US-A-4,636,385. Other specific WC strain bovine rotaviruses that may contribute the antigen originally known as v.p.3 but later as v.p.4 to the novel reassortant are WC2, WC4, WC5, WC6, WC7, WC8, WC9, and WC10.

The neutralization antigen from gene segment 4 was originally known in the literature as v.p.3. However, the work of Liu, Offit and Estes reported in the article "Identification of the simian rotavirus SA 11 genome segment 3 product" in Virology, 136, 26-32 (1988) showed that a neutralization antigen could also be derived from the gene segment 3 of rotaviruses. Those workers therefore proposed in that article that the neutralization antigen from gene segment 3 be known as v.p.3 and the name of the neutralization antigen from gene segment 4 be changed from v.p.3 to v.p.4. That nomencleture change has been followed in the present application, where the neutralization antigen in question, which is derived from gene segment 4 as mentioned elsewhere in the text, will hereinafter be referred to as the neutralization antigen v.p.4 (although without intending thereby any change in its identity from the antigen identified as v.p.3 in the US patent application from which priority under the International Convention is claimed).

The human rotavirus gene which encodes for the neutralization antigen v.p.7 in this class of the novel reassortants may be selected from any human rotavirus serotype for which immunization is desired. A non-exclusive list of such serotypes includes the serotypes 1, serotype 2, serotype 3, serotype 4, serotype M69 and serotype WI61, as well as new viral serotypes yet to be identified.

A specific example of reassortants according to this class of the invention is WC3:2-5 (otherwise WI78-1,6-11), described more fully below. This reassortant has been deposited with the American Type Culture Collection, 12301 Parklawn Drive, Rockville, Maryland, USA deposited on November 25, 1987 under accession number ATCC VR2193 and VR2195. Unless stated to the contrary, all deposits with the ATCC referred to herein, are available to the public in accordance with the requirements of Rule 28(3) and (4) EPC, availability until the publication of the mention of grant of the European patent or until the date on which the application has been refused or withdrawn or is deemed to be withdrawn being effected only by the issue of a sample to an expert nominated by the requester. Other reassortants provided by the invention are WC3:2-6 (otherwise WI78-1,7-11); WI61:7,9; WI79-4, WI78-4 and WI61-4. These reassortants, as well as other reassortants encompassed by the invention can be obtained by one skilled in the art by following the procedures disclosed herein.

Another class of novel reassortants according to the present invention contains at least the gene from a human rotavirus encoding the v.p.4 neutralization antigen and the gene from a bovine WC3 strain rotavirus encoding the v.p.7 neutralization antigen, the remainder of the genes being from the human rotavirus and/or from the bovine strain rotavirus. The same bovine WC strains may be employed in this class as were employed to contribute the v.p.4 antigen in the other class as described above. Similarly the human rotaviruses useful in this class of reassortants are as described above for the other class.

As another aspect of this invention there is provided a vaccine for providing immunological protection against acute diarrhea caused by human rotavirus which contains at least one of the novel reassortant rotaviruses of the present invention.

Another aspect of the invention involves a method for preparing a novel reassortant virus of the invention. This method involves the steps of:
(a) infecting a suitable cell substrate with a mixed infection of a bovine rotavirus strain WC3 and a human rotavirus under conditions enabling gene reassortment in the infected culture; and
(b) examining progeny clones from plaques formed in the infected culture by PAGE for the presence either of a reassortant containing at least the bovine WC3 rotavirus gene encoding the v.p.4 antigen and two human rotavirus genes one of which is the gene encoding the v.p.7 antigen or of a reassortant containing at least the bovine WC3 rotavirus gene encoding the v.p.7 antigen and the human rotavirus gene encoding the v.p.4 antigen. Suitable cell substrates for use in this method include CV-1, Vero, BSC-1, MA104 and primary primate kidney cell cultures.

Human beings may be vaccinated against human rotavirus infection employing the reassortant vaccines of the invention. The vaccination method involves administering by oral or nasal route, or by injection, to human beings at least one dose comprising from about 10^{6.0} to about 10^{9.0} pfu, of the vaccine. This method can also employ an additional dose of the vaccine about 3 to 4 weeks after the first dose. The vaccine may be administered directly to infants or to pregnant or nursing mothers for purposes of transferring immunity to an infant.

Other aspects and advantages of the present invention will be apparent upon consideration of the following detailed description of the invention, including illustrative examples of the practice thereof.

### Detailed Description of the Invention

This invention involves rotavirus reassortants suitable for use as vaccines to confer immune protection against human rotavirus infection. The reassortants are produced by genetic reassortment between an attenuated bovine rotavirus WC-3, which in a first class of reassortants contributes at least the gene segment encoding the v.p.4 protein and in a second class of reassortants contributes at least the gene segment encoding the v.p.7 protein, and a rotavirus representing an epidemiologically important human serotype, which contributes to the first class of reassortants at least the gene segment encoding the v.p.7 protein. and at least one other gene segment and to the second class of reassortants at least the gene segment encoding the v.p.4 protein.

The protein v.p.4 is an 88,000 dalton major surface structural protein product of gene 4 of a rotavirus. Like v.p.7, it functions as a major serotype-specific antigen, operative in SN tests, capable of inducing serotype-specific neutralizing antibody, and capable in a mouse system of inducing serotype specific immune protection against rotavirus disease. [See, Offit et al, (1986) supra]. In experimental studies v.p.4 has been shown to also play a role in the control of rotavirus host range in cell culture [Kalica et al, Virol., 125:194 (1983); Greenberg et al, Infec. Immunol., 37:104 (1982)], and in the mediation of rotavirus virulence in vivo [Offit et al, J. Virol., 57:46 (1986)].

Most desirably, a reassortant according to the present invention contains gene 4, encoding v.p.4, from the attenuated bovine rotavirus strain WC3 or its progeny described in detail in US-A-4,636,385; the disclosures of that patent are incorporated by reference herein to provide additional information about this rotavirus strain. Representative isolates of this strain type which may be substituted for WC3 are WC2, WC4, WC5, WC6, WC7, WC8, WC9 and WC10. These bovine rotaviruses are readily distinguishable from other strains of bovine rotavirus by their distinctive RNA electropherotype, their failure to hemagglutinate primate red blood cells, their plaque morphology and response in the SN test. The presently most preferable strain for use in the reassortants of the present invention is WC3. WC3 replicates to a high titer in CV-1 cells (ATCC CCL70) and is known to be attenuated and immunogenic in human infants [Clark et al, Amer. J. Dis. Children, 140:350 (1986)].

When the v.p.4-encoding segment is contributed by bovine WC3 rotavirus or an isolate thereof, the rotavirus contributing the v.p.7 protein-encoding gene segment may be any selected human serotype virus, including both the known serotypes 1, 2, 3, 4, and M69, and a new serotype WI61 [See, Clark et al, 1987 supra]. The selected human rotavirus may also be attenuated, if desired, for use in the reassortant. The v.p.7 protein is coded for by either gene segment 8 or gene segment 9 of the particular human rotavirus. The location of the v.p.7 encoding gene may be determined for each specific rotavirus by conventional experimental methods. Among the human rotavirus strains useful in the present invention are strains WI79, WICC1, WI/U of serotype 1; strains WI-SC2 and WI/Q of serotype 2; strains WI77, WI78, WI/P, and WIC-17 of serotype 3; strain WI-CC4 of serotype 4; and serotype/strain WI61. This list of human serotype rotaviruses is non-exclusive; newly identified human origin rotaviruses are also expected to be useful in the methods and compositions disclosed herein. The human rotavirus contributes at least one other gene and may contribute more than one other gene in addition the v.p.7 encoding gene to the reassortant. However, when the v.p.7-encoding gene is contributed by human rotavirus the v.p.4-encoding gene must be contributed by the bovine strain, to provide reassortants according to the invention.

Specific examples of reassortants of the present invention are a novel reassortant, designated WC3:2-5 (otherwise WI78-1,6-11), which contains four genes, 2 through 5, from bovine rotavirus WC3 and seven genes, 1, and 6 through 11 of human serotype 3 rotavirus strain WI78. Similarly, novel reassortant WC3:2-6 (otherwise WI78-1,7-11) contains five genes, 2 through 6, from bovine rotavirus WC3 and six genes, 1, and 7 through 11, from human serotype 3 rotavirus strain WI78.

Additional exemplary novel reassortants utilize human serotype WI61 as the contributor of the v.p.7 protein encoding gene. For example, WI61:7,9 contains genes 7 and 9 from the new human serotype strain WI61 and genes 1 through 6, 8, 10 and 11 from WC3.

Additional reassortants provided by the present invention contain the gene encoding the v.p.4 protein contributed by an attenuated human rotavirus and the gene encoding the v.p.7 protein contributed by attenuated bovine rotavirus WC3. Examples of novel reassortants according to this aspect of the invention are WI79-4 which contains gene 4 encoding the v.p.4 protein from human serotype 1, strain WI79 and genes 1 to 3, and 5 through 11 from bovine strain WC3. Another such novel reassortant is WI78-4, which contains gene 4 from human serotype 3 strain WI78 and the remaining ten genes from WC3. Novel reassortant WI61-4 similarly contains gene 4 from the new human serotype rotavirus and the remaining genes from WC3.

One of the novel reassortants of the present invention is described in detail below. However, all of the above-described reassortants, as well as additional reassortants which are not specifically identified may be prepared by one of skill in the art employing the methods of the present invention, as described in the examples.

Also included in this invention is a method for producing the novel reassortants. This method include the step of isolating the human and other species rotavirus by culturing in a suitable cell culture. The isolation technique is standard and is described in more detail in Example 1.

Suitable cells for such isolation and infection include African green monkey kidney cells CV-1 (ATCC CCL-70); BSC-1 (ATCC CCL-26), fetal green monkey cell MA-104 and VERO (ATCC CCL-81), and primary primate kidney cell cultures. For purposes of this invention, primary primate kidney cell cultures include first, second (secondary) or third (tertiary) passages of kidney cells derived from the indicated species of primate. Each of these cell culture substrates may be grown in BHK medium [MacPherson, I and M. Stoker, Virology, 16:147 (1962)], supplemented with 10% fetal calf serum, Eagle's minimal essential medium with 10% fetal calf serum, or medium 199 with 10% fetal calf serum. These media may also contain gentamicin, 25 micrograms per milliliter. These cell lines may be used alone, or in combination in serial passaging of the viruses. When used in combination, a separate but different cell line can be used in each of the various passages of the virus.

Secondly, a suitable cell culture is infected with both the attenuated bovine rotavirus strain WC3 and the desired human serotype rotavirus. Mixed infections are designed to maximize the potential for reassortment by ensuring that large and equal concentrations of each parent virus are replicating simultaneously. After infection and sufficient time and conditions for gene reassortment, reassortant progeny clones are examined by random selection of plaques, e.g., by performing a plaque assay of the virus yield from the mixed infection. The virus is propagated in individual plaques which are induced by inoculation of the yield of the mixed infection onto another cell culture monolayer. Page-SS analysis of each such virus population is performed to compare its electropherotype with that of each parental rotavirus. The proportion of reassortant rotaviruses isolated may be enhanced by selecting plaques whose morphology differs from that of either parent. Alternatively, progeny clones may be selected from the virus yield of the mixed infection after treatment with hyperimmune antiserum to the serotype of the rotavirus contributing the v.p.4 encoding gene [See, e.g., the method of US-A-4,571,385], prior to performing the plaque analysis of the population. This method may be applied to any human or animal virus for which a serotype-specific vaccine is required.

Progeny clones are examined by harvesting individual plaques, which are then cultivated individually in cell culture and examined for their gene constitution by polyacrylamide gel electrophoresis with silver stain (PAGE-SS) according to the procedure of Dolan et al, J. Clin. Microbiol. 21:753 (1985). Reassortant progeny clones are selected as vaccine condidates if their PAGE-SS reveals the presence of at least gene 4, encoding v.p.4 antigen, from the attenuated bovine rotavirus strain WC3 and the gene coding for the major rotavirus surface antigen v.p.7 associated with virus-neutralization and at least one other gene from the rotavirus against which immune protection is being sought. Alternatively, for another set of reassortants according to the invention, the clones are selected as vaccine candidates if the PAGE-SS reveals the presence of gene 4 from the human serotype rotavirus and the gene encoding v.p.7 from the other species rotavirus, e.g., WC3.

The invention also includes vaccines for providing immunological protection against acute diarrhea caused by human rotavirus infection. These vaccines contain one or more of the novel reassortants of the present invention and, optionally, conventional vaccine adjuvants and/or carriers, e.g., aqueous suspensions of aluminum and magnesium hydroxides. The method of preparing a vaccine according to the invention involves inoculation of a suitable cell substrate, e.g., CV-1 cells, and passaging of the reassortant therein. By combining one or more different human serotype reassortants, the vaccine can elicit a polytypic viral neutralizing antibody response.

The vaccine preparations of the invention may be used in a method of vaccinating human beings against human or bovine rotavirus infection. The vaccine preparations including one or more of the reassortants described herein are administered, preferably by oral or nasal route, in high dose. The vaccine may also be administered by injection.

Alternatively, the vaccine may be administered to pregnant or nursing mothers as a means for transferring immunity to the infant. The dosage for all routes of administration is generally greater than 10⁶, and preferably between 10⁶ and 10⁹ plaque forming units (pfu) of the reassortant. Additional doses of the vaccines may also be administered. The preparation of a pharmaceutically acceptable vaccine, having due regard to pH, isotonicity, stability and the like, is within the skill of the art. The dosage regimen involved in a method for vaccination will be determined considering various hosts and environmental factors, e.g. the age of the patient, time of administration and the geographical location and environment.

The following examples demonstrate the methods and compositions of the present invention, including two specific exemplary reassortant viruses.

### Example 1: Isolation of the Rotaviruses

The bovine rotavirus strain WC3 and human rotavirus strains used in producing reassortants according to the invention were isolated in cell line MA104 and then adapted to growth in cell line CV-1.

The human origin rotaviruses were isolated by standard techniques as described previously for isolation of human rotavirus strain WI61 in Clark et al. (1987) supra. Stools of infants ill with gastroenteritis were determined to contain rotavirus by means of examination by the PAGE-SS technique for detection of the rotavirus-characteristic 11 segments of double-stranded RNA. Rotavirus-containing stools were emulsified into a 5% (w/v) suspension in serum-free Eagle's Minimal Essential Medium containing 500 units of penicillin/ml, 500 micrograms of streptomycin/ml, 40 micrograms of gentamicin/ml 50 units of nystatin/ml, and 20 micrograms of trypsin/ml. The stool suspension was clarified by centrifugation at 2000Xg for 30 minutes. Clarified supernatant fluid was incubated with an equal volume of purified trypsin (10 microgram/ml) in phosphate buffered saline (PBS) for 60 minutes at 37 degrees C. The trypsin-treated stool supernatant fluid was inoculated in a volume of 0.2 ml into tube cultures of MA104 cells which had previously been washed three times with PBS. After absorption of this rotavirus-containing fluid for 30 minutes at 37 degrees, the tube cultures were fed with 1.5 ml of Sato medium containing 1 microgram/ml of purified trypsin and incubated in a roller apparatus at 37 degrees.

Inoculated cell cultures were harvested after seven days of incubation by freezing and thawing of the combined cells and cell culture medium. Serial passage was accomplished by inoculating 0.2 ml of undiluted cell culture suspension into fresh tubes of MA104 cell culture treated in the same manner as the initial passage inoculated with stool suspension supernatant fluid. Cell culture suspensions from each successive passage were analyzed for the presence of rotavirus RNA by the PAGE-SS technique. Detectable concentrations of rotavirus RNA were usually obtained by the second or third passage level. Visible cytopathic effect (CPE) usually appeared by the second to fifth cell culture passage. After the rotavirus strain has become cytopathic, serial passages were made whenever CPE involved more than 75% of the cell monolayer (2 to 7 days). When a rotavirus isolate consistently induced CPE in roller tube cultures within 48 hours (usually within 4 to 8 passages), serial passage was performed in stationary cultures of MA104 cells fed with BHK medium supplemented with 13 micrograms/ml of unpurified trypsin (Flow Labs). Serial subculture in MA104 cell stationary cultures was performed in the same manner as that used for roller tubes, and was continued until the isolated rotavirus was determined to efficiently induce plaques under agarose overlay in MA104 cell culture.

When the rotavirus isolate efficiently induces plaques in the plaque induction assay according to Offit et al., J. Virol. Methods, 7:29 (1983) [usually 10⁵ to 10⁷ pfu per ml], it has adapted to growth in the MA104 cell culture. It is then adapted to growth in stationary cultures of CV-1 cells by similar serial passage methods, except that the medium is Eagle's MEM serum-free and containing 6.25 microgram/ml unpurified trypsin (Flow). At varying passage levels, as appropriate, the isolated rotavirus may be genetically purified by isolation and propagation of a single plaque produced in MA104 cell culture. Mechanical aspiration of cells within a single plaque, well separated from any surrounding plaques is followed by serial propagation of virus contained in this cell suspension by standard technique.

The identity of the cell culture-adapted rotavirus compared with the virus in the original stool suspension is confirmed by comparison of the RNA electropherotypes induced in polyacrylamide gel. The serotype of each cell culture-adapted rotavirus may be determined by reaction with serotype-specific hyperimmune antisera to prototype rotaviruses prepared in rabbits or guinea pigs [Clark et al, (1987) supra.].

### Example 2: Producing the Reassortant WC3:2-5

MA104 cell culture in a 24 well plastic plate (approximately 1 cm² of cell monolayer per well) was washed twice with PBS and inoculated with a mixture containing 1.5 X 10⁵ pfu of human serotype 3 rotavirus strain WI78 (passage level 17) and 1.5 X10³ pfu of WC3 rotavirus (passage level 11). An excess of the WI78 was employed because WC3 grows more rapidly than most human-origin rotavirus isolates. The virus was allowed to absorb to the cells by incubation for 30 minutes at 37 degrees C, after which 1.5 ml/well BHK medium with 13 micrograms/ml trypsin/ml was added and incubation was continued at the same temperature. When CPE involved the entire cell population at 72 hours post infection, the culture was harvested by three cycles of freezing and thawing and the progeny virus was plaqued by standard technique in MA104 cells. Plaques that were smaller than those induced by parental rotavirus WC3 were harvested, propagated, and analyzed by PAGE-SS.

Among the progeny plaques of this mixed infection was a reassortant designated WI78:1,7,9. This reassortant contained gene segments 1, 7 and 9 of parent rotavirus WI78 and all other gene segments from WC3. This virus was serially passaged 10 times in MA104 cells including three plaque purifications. Because WI79:1,7,9 was not neutralized by reference hyperimmune antiserum to serotype 3 rotavirus [see, e.g., Clark et al, AJDC, (1986) supra], it was observed that gene 8 of parent WI78, not gene 9, must code for the serotype 3-specific v.p.7 protein.

A second mixed infection was performed as above, with the WI78:1,7,9 reassortant mixed with WI78 rotavirus. Among the progeny plaques derived from this mixed infection was a reassortant which contained genes 2 through 5 from bovine rotavirus WC3 and all remaining genes from WI78 rotavirus. This reassortant, designated WC3:2-5, was passaged six times in MA104 cell culture, including three plaque purifications. Reaction of WC3:2-5 virus in a virus neutralization (VN) test with hyperimmune reference sera to both human serotype 3 rotavirus and to bovine rotavirus serotype virus indicated that its phenotype is both serotype 3 and bovine.

WC3:2-5 also replicates to a titer of at least 10^{6.0} pfu/ml in CV-1 cells. At this dose, it is entirely attenuated for orally inoculated adults and for infants as young as two months of age. A high percentage of infants orally inoculated with WC3:2-5 respond, with approximately equal frequency, with virus neutralizing (VN) antibody to human serotype 3 rotavirus and/or bovine serotype rotavirus.

### Example 3: Method for Making Exemplary Novel Vaccines from WC3:2-5

WC3:2-5 reassortant was adapted to growth in CV-1 cell culture by four additional passages in CV-1 cells. The fourth CV-1 cell passage comprises a test vaccine evaluated in human adult volunteers and in infants.

The vaccine was produced by inoculation of 850 cm² plastic roller bottles of CV-1 cell culture grown in Eagle's MEM medium containing 10% fetal calf serum and 25 micrograms/ml gentamicin. The cells were inoculated when a confluent monolayer was obtained six days after cell seeding. The cell cultures were washed twice with PBS to remove residual serum and inoculated with 10 ml of WC3:2-5 rotavirus stock containing a total of 6.0 X 10⁶ pfu (multiplicity of infection MOI was approximately 0.10). The virus was allowed to absorb to the cells by incubation at 37°C for 60 minutes. The inoculum was removed and the cells were fed with 80 ml per roller flask of BHK medium serum-free and containing 5.0 microgram/ml of unpurified trypsin and 25 microgram/ml of gentamicin. The rotavirus-infected cell cultures were incubated at 37° until the entire monolayer exhibited CPE, approximately 72 hours. The virus was then harvested by disrupting the cells with three cycles of freezing and thawing. Cell debris was removed by centrifugation at 2000Xg for 60 minutes at 4°C. The resulting supernatant fluid comprised the test vaccine. It was frozen at -70°C pending testing for sterility, freedom from adventitious viruses, and assay of concentration of infectious reassortant rotavirus.

Sterility tests consisted of inoculation of the vaccine into standard laboratory media for the culture of aerobic and anaerobic bacteria, mycobacteria, and fungi. The vaccine was tested for mycoplasma by inoculation of 3T3 mouse cells in culture, followed by staining with Hoechst stain for intracytoplasmic DNA. Testing for adventitious viruses included inoculation of human and primate cell cultures in the presence of serotype-specific anti-rotavirus serum obtained by conventional methods, to suppress the replication of vaccine virus, which were observed for the appearance of CPE and/or hemadsorption. Adult and newborn mice were inoculated intracerebrally and orally with the vaccine and observed subsequently for 30 days. Adult guinea pigs were inoculated intraperitoneally and observed for 15 days post-inoculation.

Infectious reassortant rotavirus WC3:2-5 concentration was determined by standard plaque assay. The vaccine in this example had a titer of 10^{6.3} pfu/ml. The vaccine stock has been deposited with the American Type Culture Collection, as ATCC No. VR2195.

### Example 4: Administration of Novel Vaccine containing WC3:2-5

Administration of vaccine to adults: A full dose (10^{6.3} pfu) of WC3:2-5 was administered orally to seven normal adult volunteers of age 31 to 50 years. Volunteers were given 30 ml of Maalox (Rorer) prior to vaccine, to buffer stomach acids. All remained clinically normal for 30 days. None was observed to be shedding vaccine rotavirus in feces three days after inoculation. One adult exhibited an increase in serum antibody to both WC3 rotavirus and to human serotype 3 rotavirus at 30 days post-inoculation. The other volunteers, who were all previously seropositive to rotavirus of serotype 1 and/or 3 and/or bovine serotype, did not exhibit a rise in serum antibody to serotype 3 or bovine serotype rotavirus. No volunteer exhibited a rise in antibody to serotype 1 rotavirus.

Administration of vaccine to infants: In a cautious series of vaccine trials, WC3:2-5 was first administered in a reduced dose of 10^{5.3} pfu to two infants who had been previously immunized with WC3 vaccine, then to two infants given WC3:2-5 vaccine as an original dose of 10^{4.3} pfu and then to two infants given an original dose of 10^{5.3} pfu. Finally, 24 infants were given a full dose of 10^{6.3} pfu. All infants were given vaccine orally in a dose of 2.5 ml containing 20% cherry syrup. In approximately one half of the infants stomach acids were neutralized by oral administration of at least 30 ml of infant formula, no more than 30 minutes prior to vaccine administration.

All infants were monitored for signs of vaccine-associated illness for the first seven days post-inoculation, but no symptoms were observed. A fecal sample was collected between three and seven days post inoculation for detection of vaccine rotavirus by standard plaque assay: a single infant shed vaccine rotavirus in low titer (less than 10^{3.0} pfu/gram feces) after receiving a vaccine dose of 10^{5.3} pfu. Twelve of 29 (41%) of the infants who completed the 30 day trial exhibited an increase in serum antibody at 30 days post infection when sera were tested against bovine rotavirus strain WC3 and prototype rotavirus strains SA11 (serotype 3) and Wa (serotype 1). The observed serum antibody responses were most frequently directed towards the bovine serotype WC3 rotavirus (8 of the 29 infants, or 28%) and the serotype 3 rotavirus strain SA11 (9 of the 29 infants, or 31%), reflecting the bivalent serotype 3 and bovine serotype constitution of the WC3:2-5 reassortant rotavirus. Only 4 infants, or 14% demonstrated SN response to Wa.

Analysis of the WC3:2-5 reassortant virus-vaccinated population (only the 24 infants given a full dose of 10^{6.3} pfu) indicated that infants 5 to 11 months old responded more efficiently (6/11, or 55%), than those 2 to 4 months old (3/13, or 23%). Infants with low titers of serum antibody prior to administration of vaccine were also more likely to exhibit an immune response to vaccine. For example, in the population of infants 5 to 11 months old possessing serum antibody titers of less than 1:250 to serotype 3 rotavirus prior to vaccine, 6 of 8, or 75%, developed a rise in serum antibody in response to vaccine.

The percentage of infants developing an immune response could also be enhanced by administration of a second dose of WC3:2-5 vaccine given orally 30 days after the original dose. Therefore, of 19 infants given two full doses, seven of nineteen, or 37%, responded to the first dose; 10 of 19, or 53%, responded to the second booster dose, and 13 of 19, or 68%, responded to either the first, second or both doses.

Antigenic variation between different rotavirus strains classified within serotype 3 has been observed, when compared in cross neutralization tests using hyperimmune [Hoshino et al, 1984] or monoclonal [Taniguchi et al, 1985] antisera. Therefore, the sera collected from 18 infants 30 days after their first dose of WC3:2-5 reassortant virus were tested for neutralization antibody to WC3:2-5 reassortant as well as to the SA11 prototype serotype 3 rotavirus. Many infants developed an immune response to WC3:2-5 reassortant virus in the absence of a detectable serum antibody response to SA11 virus. After a single dose of WC3:2-5 vaccine, the combined incidence of infants with a serum antibody response to either of the serotype 3 viruses was 13 of 18, or 72%.

## Claims (Claims for the following Contracting State(s): AT, BE, CH, DE, FR, GB, IT, LI, LU, NL, SE)

1. A reassortant rotavirus useful as a component of a vaccine against human rotavirus infection comprising:
(a) the gene segment 4, encoding the v.p.4 neutralization antigen, from a bovine WC3 strain rotavirus or an isolate thereof, which isolate is characterised by the WC3 characteristics of RNA electropherotype, failure to hemagglutinate primate red blood cells, plaque morphology, and response in the serum neutralization test; and
(b) at least two gene segments from a human rotavirus, wherein one of said at least two gene segments is the gene segment encoding the v.p.7 neutralization antigen; said reassortant being further characterised by its remaining rotavirus gene segments originating solely from said human strain, solely from said bovine strain or from both said human and said bovine strains and said reassortant being further characterised as capable of inducing a protective immune response in human adult and infant patients to challenge with native rotavirus without producing serious adverse effects in said patients.

2. The reassortant according to claim 1, wherein said human rotavirus is selected from the group consisting of virus serotype 1, serotype 2, serotype 3, serotype 4, serotype M69, and serotype WI61.

3. The reassortant according to claim 1, wherein said human gene segment encoding the v.p.7 neutralization antigen is gene segment 9.

4. The reassortant according to claim 3, wherein said reassortant is WI61-7,9, said reassortant characterised by bovine strain WC3 rotavirus gene segments 1-6, 8 and 10-11 and human strain WI61 gene segments 7 and 9.

5. The reassortant according to claim 1, wherein said human gene segment encoding the v.p.7 neutralization antigen is gene segment 8.

6. The reassortant according to claim 5, wherein said reassortant is WI78-1,6-11, said reassortant consisting of bovine strain WC3 rotavirus gene segments 2-5 and human strain WI78 rotavirus gene segments 1 and 6-11.

7. The reassortant according to claim 5, wherein said reassortant is WI78-1,7-11, said reassortant characterised by bovine strain WC3 rotavirus gene segments 2-6 and human strain WI78 rotavirus gene segments 1 and 7-11.

8. A reassortant rotavirus useful as a component of a vaccine against human rotavirus infection comprising
(a) a gene segment contributed by a bovine WC3 strain rotavirus or an isolate thereof, which isolate is characterised by the WC3 characteristics of RNA electropherotype, failure to hemagglutinate primate red blood cells, plaque morphology and response in the serum neutralization test, said gene segment being the gene segment encoding the v.p.7 neutralization antigen; and
(b) at least the gene segment encoding the v.p.4 neutralization antigen from the human rotavirus, said reassortant being further characterised by its remaining rotavirus gene segments originating solely from said human strain, solely from said bovine strain or from both said human and said bovine strains and said reassortant being further characterised as capable of inducing a protective immune response in human adult and infant patients to challenge with native rotavirus without producing serious adverse effects in said patients.

9. A reassortant rotavirus useful as a component of a vaccine against human rotavirus infection according to claim 8 consisting of the gene segment encoding the v.p.4 neutralization antigen from a human rotavirus and ten gene segments contributed by a bovine WC3 strain rotavirus or an isolate thereof, which isolate is characterised by the WC3 characteristics of RNA electropherotype, failure to hemagglutinate primate red blood cells, plaque morphology, and response in the serum neutralization test, said reassortant being further characterised as capable of inducing a protective immune response in human adult and infant patients to challenge with native rotavirus without producing serious adverse effects in said patients.

10. The reassortant according to claim 8 wherein said human rotavirus is selected from the group consisting of virus serotype 1, serotype 2, serotype 3, serotype 4, serotype M69, and serotype WI61.

11. The reassortant according to claim 8 selected from the group consisting of
WI79-4, said reassortant characterised by bovine strain WC3 rotavirus gene segments 1-3 and 5-11 and human strain WI79 rotavirus gene segment 4;
WI78-4, said reassortant characterised by bovine strain WC3 rotavirus gene segments 1-3 and 5-11 and human strain WI78 rotavirus gene segment 4; and
WI61-4, said reassortant characterised by bovine strain WC3 rotavirus gene segments 1-3 and 5-11 and human strain WI61 rotavirus gene segment 4.

12. A vaccine capable of safe use in humans for immunization against human rotavirus infection comprising a pharmaceutical carrier containing at least one reassortant rotavirus selected from the group consisting of:
(a) a reassortant rotavirus according to claim 1; and
(b) a reassortant rotavirus according to claim 8.

13. A vaccine according to claim 12 for administration to human beings by the oral or nasal route or by injection, the vaccine being in the form of a unit dose comprising from about 10^{6.0} to about 10^{9.0} pfu.

14. A method for preparing a reassortant virus according to claim 1 or claim 8 comprising the steps of:
(a) infecting a suitable cell substrate with a mixed infection of a bovine rotavirus strain WC3 and a human rotavirus under conditions enabling gene reassortment in said infected culture;
(b) examining progeny clones from plaques produced in said culture by PAGE for the presence either of a reassortant containing at least the bovine rotavirus WC3 gene segment encoding the v.p.4 antigen and two human rotavirus genes, where one of said two gene segments is the gene segment encoding the v.p.7 antigen, or of a reassortant containing at least the human rotavirus gene segment encoding the v.p.4 antigen and the bovine rotavirus WC3 gene segment encoding the v.p.7 antigen.

15. The method according to claim 14 wherein said cell substrate is selected from the group consisting of CV-1, Vero, BSC-1, MA104 and primary primate kidney cell cultures.

## Claims (Claims for the following Contracting State(s): ES, GR)

1. A method for preparing a reassortant rotavirus useful as a component of a vaccine against human rotavirus infection, said reassortant rotavirus comprising:
(a) the gene segment 4, encoding the v.p.4 neutralization antigen, from a bovine WC3 strain rotavirus or an isolate thereof, which isolate is characterised by the WC3 characteristics of RNA electropherotype, failure to hemagglutinate primate red blood cells, plague morphology, and response in the serum neutralization test; and
(b) at least two gene segments from a human rotavirus, wherein one of said at least two gene segments is the gene segment encoding the v.p.7 neutralization antigen; said reassortant rotavirus being further characterised by its remaining rotavirus gene segments originating solely from said human strain, solely from said bovine strain or from both said human and said bovine strains and said reassortant rotavirus being further characterised as capable of inducing a protective immune response in human adult and infant patients to challenge with native rotavirus without producing serious adverse effects in said patients,
said method for preparing comprising the steps of:
(a) infecting a suitable cell substrate with a mixed infection of a bovine rotavirus strain WC3 and a human rotavirus under conditions enabling gene reassortment in said infected culture;
(b) examining progeny clones from plagues produced in said culture by PAGE for the presence of a reassortant containing at least the bovine rotavirus WC3 gene segment encoding the v.p.4 antigen and two human rotavirus genes, where one of said two gene segments is the gene segment encoding the v.p.7 antigen.

2. A method for preparing a reassortant rotavirus useful as a component of a vaccine against human reassortant rotavirus infection, said rotavirus comprising
(a) a gene segment contributed by a bovine WC3 strain rotavirus or an isolate thereof, which isolate is characterised by the WC3 characteristics of RNA electropherotype, failure to hemagglutinate primate red blood cells, plague morphology and response in the serum neutralization test, said gene segment being the gene segment encoding the v.p.7 neutralization antigen; and
(b) at least the gene segment encoding the v.p.4 neutralization antigen from a human rotavirus, said reassortant rotavirus being further characterised by its remaining rotavirus gene segments originating solely from said human strain, solely from said bovine strain or from both said human and said bovine strains and said reassortant rotavirus being further characterised as capable of inducing a protective immune response in human adult and infant patients to challenge with native rotavirus without producing serious adverse effects in said patients,
said method for preparing comprising the steps of;
(a) infecting a suitable cell substrate with a mixed infection of a bovine rotavirus strain WC3 and a human rotavirus under conditions enabling gene reassortment in said infected culture;
(b) examining progeny clones from plagues produced in said culture by PAGE for the presence of a reassortant containing at least the human rotavirus gene segment encoding the v.p.4 antigen and the bovine rotavirus WC3 gene segment encoding the v.p.7 antigen.

3. A method according to claim 1 or 2, wherein said cell substrate is selected from the group consisting of CV-1, Vero, BSC-1, MA104 and primary primate kidney cell cultures.

4. A method according to any of claims 1 to 3, wherein said human rotavirus is selected from the group consisting of virus serotype 1, serotype 2, serotype 3, serotype 4, serotype M69, and serotype WI61.

5. A method according to claim 1 or to claim 3 or 4 appendant to claim 1, wherein said human gene segment encoding the v.p.7 neutralization antigen is gene segment 9.

6. A method according to claim 5, wherein said reassortant is WI61-7,9, said reassortant characterised by bovine strain WC3 rotavirus gene segments 1-6, 8 and 10-11 and human strain WI61 gene segments 7 and 9.

7. A method according to claim 1 or to claim 3 or 4 appendant to claim 1, wherein said human gene segment encoding v.p.7 neutralization antigen is gene segment 8.

8. A method according to claim 7, wherein said reassortant is WI78-1,6-11, said reassortant consisting of bovine strain WC3 rotavirus gene segments 2-5 and human strain WI78 rotavirus gene segments 1 and 6-11.

9. A method according to claim 7, wherein said reassortant is WI78-1,7-11, said reassortant characterised by bovine strain WC3 rotavirus gene segments 2-6 and human strain WI78 rotavirus gene segments 1 and 7-11.

10. A method according to claim 2 or to claim 3 or 4 appendant to claim 2, wherein said reassortant is selected from the group consisting of
WI79-4, said reassortant characterised by bovine strain WC3 rotavirus gene segments 1-3 and 5-11 and human strain WI79 rotavirus gene segment 4;
WI78-4, said reassortant characterised by bovine strain WC3 rotavirus gene segments 1-3 and 5-11 and human strain WI78 rotavirus gene segment 4; and
WI61-4, said reassortant characterised by bovine strain WC3 rotavirus gene segments 1-3 and 5-11 and human strain WI61 rotavirus gene segment 4.

## Patentansprüche (Patentansprüche für folgende(n) Vertragsstaat(en): AT, BE, CH, DE, FR, GB, IT, LI, LU, NL, SE)

1. Reassortantes Rotavirus zur Verwendung als Bestandteil einer Vakzine gegen Infektion mit Human-Rotavirus, umfassend:
(a) den von einem Rinder-Rotavirusstamm WC3 oder einem seiner Isolate stammenden, für das Neutralisierungsantigen v.p.4 kodierenden Genabschnitt 4, wobei dieses Isolat durch die WC3-Merkmale RNA-Elektropherotyp, Unfähigkeit zur Hämagglutination roter Blutkörperchen von Primaten, Plaque-Morphologie und Antwort im Serum-Neutralisationstest gekennzeichnet ist; sowie
(b) zumindest zwei Genabschnitte eines Human-Rotavirus, wobei es sich bei einem dieser mindestens zwei Genabschnitte um den für das Neutralisierungsantigen v.p.7 kodierenden Genabschnitt handelt;
wobei diese Reassortante weiterhin durch ihre zurückbleibenden, ausschließlich von diesem Humanstamm, ausschließlich von diesem Rinderstamm oder von sowohl diesem Human- als auch diesem Rinderstamm stammenden Rotavirus-Genabschnitte gekennzeichnet ist und diese Reassortante weiterhin durch die Fähigkeit gekennzeichnet ist, im menschlichen Patienten in Erwachsenen- bzw. Kindesalter eine schützende Immunantwort auf Provokation mit nativem Rotavirus zu induzieren, ohne daß bei diesen Patienten schwerwiegende Nebenwirkungen auftreten.

2. Reassortante nach Anspruch 1, wobei dieses Human-Rotavirus aus der Gruppe bestehend aus den Viren des Serotyps 1, Serotyps 2, Serotyps 3, Serotyps 4, Serotyps M69 und Serotyps WI61 ausgewählt ist.

3. Reassortante nach Anspruch 1, wobei es sich bei dem für das Neutralisierungsantigen v.p.7 kodierenden Human-Genabschnitt um den Genabschnitt 9 handelt.

4. Reassortante nach Anspruch 3, wobei es sich bei dieser Reassortante um WI61-7,9 handelt und diese Reassortante durch die Genabschnitte 1-6, 8 und 10-11 des Rinder-Rotavirusstamms WC3 und die Genabschnitte 7 und 9 des Humanstamms WI61 gekennzeichnet ist.

5. Reassortante nach Anspruch 1, wobei es sich bei dem für das Neutralisierungsantigen v.p.7 kodierenden Genabschnitt um den Genabschnitt 8 handelt.

6. Reassortante nach Anspruch 5, wobei es sich bei dieser Reassortante um WI78-1, 6-11 handelt und diese Reassortante aus den Genabschnitten 2-5 des Rinder-Rotavirusstamms WC3 und den Genabschnitten 1 und 6-11 des Human-Rotavirusstamms WI78 besteht.

7. Reassortante nach Anspruch 5, wobei es sich bei dieser Reassortante um WI78-1, 7-11 handelt und diese Reassortante durch die Genabschnitte 2-6 des Rinder-Rotavirusstamms WC3 und die Genabschnitte 1 und 7-11 des Human-Rotavirusstamms WI78 gekennzeichnet ist.

8. Reassortantes Rotavirus zur Verwendung als Bestandteil einer Vakzine gegen Infektion mit Human-Rotavirus, umfassend
(a) einen aus einem Rinder-Rotavirusstamm WC3 oder einem seiner Isolate stammenden Genabschnitt, wobei dieses Isolat durch die WC3-Merkmale RNA-Elektropherotyp, Unfähigkeit zur Hämagglutination roter Blutkörperchen von Primaten, Plaque-Morphologie und Antwort im Serum-Neutralisationstest gekennzeichnet ist und es sich bei diesem Genabschnitt um den für das Neutralisierungsantigen v.p.7 kodierenden Genabschnitt handelt; sowie
(b) zumindest den für das Neutralisierungsantigen v.p.4 kodierenden Genabschnitt aus dem Human-Rotavirus, wobei diese Reassortante weiterhin durch ihre zurückbleibenden, ausschließlich von diesem Humanstamm, ausschließlich von diesem Rinderstamm oder von sowohl diesem Human- als auch diesem Rinderstamm stammenden Rotavirus-Genabschnitte gekennzeichnet ist und diese Reassortante weiterhin durch die Fähigkeit gekennzeichnet ist, in menschlichen Patienten im Erwachsenen- bzw. Kindesalter eine schützende Immunantwort auf Provokation mit nativem Rotavirus zu induzieren, ohne daß bei diesen Patienten schwerwiegende Nebenwirkungen auftreten.

9. Reassortantes Rotavirus zur Verwendung als Bestandteil einer Vakzine gegen Infektion mit Human-Rotavirus nach Anspruch 8, bestehend aus dem von einem Human-Rotavirus stammenden, für das Neutralisierungs-antigen v.p.4 kodierenden Genabschnitt sowie zehn von einem Rinder-Rotavirusstamm WC3 oder einem seiner Isolate stammenden Genabschnitten, wobei dieses Isolat durch die WC3-Merkmale RNA-Elektropherotyp, Unfähigkeit zur Hämagglutination roter Blutkörperchen von Primaten, Plaque-Morphologie und Antwort im Serum-Neutralisationstest gekennzeichnet ist und diese Reassortante weiterhin durch die Fähigkeit gekennzeichnet ist, in menschlischen Patienten im Erwachsenen- bzw. Kindesalter eine schützende Immunantwort auf Provokation mit nativem Rotavirus zu induzieren, ohne daß bei diesen Patienten schwerwiegende Nebenwirkungen auftreten.

10. Reassortante nach Anspruch 8, wobei das Human-Rotavirus aus der Gruppe bestehend aus den Viren des Serotyps 1, Serotyps 2, Serotyps 3, Serotyps 4, Serotyps M69 und Serotyps WI61 ausgewählt ist.

11. Reassortante nach Anspruch 8, ausgewählt aus der Gruppe bestehend aus
WI79-4, wobei diese Reassortante durch die Genabschnitte 1-3 und 5-11 des Rinder-Rotavirusstamms WC3 und den Genabschnitt 4 des Human-Rotavirusstamms WI79 gekennzeichnet ist;
WI78-4, wobei diese Reassortante durch die Genabschnitte 1-3 und 5-11 des Rinder-Rotavirusstamms WC3 und den Genabschnitt 4 des Human-Rotavirusstamms WI78 gekennzeichnet ist; und
WI61-4, wobei diese Reassortante durch die Genabschnitte 1-3 und 5-11 des Rinder-Rotavirusstamms WC3 und den Genabschnitt 4 des Human-Rotavirusstamms WI61 gekennzeichnet ist.

12. Vakzine zur gefahrlosen Verwendung am Menschen zur Immunisierung gegen Infektion mit Human-Rotavirus, umfassend einen pharmazeutischen Träger, der zumindest ein reassortantes Rotavirus enthält, das aus der aus:
(a) einem reassortanten Rotavirus nach Anspruch 1; und
(b) einem reassortanten Rotavirus nach Anspruch 8 bestehenden Gruppe ausgewählt ist.

13. Vakzine nach Anspruch 12 zur Verabreichung an den Menschen auf oralem oder nasalem Weg bzw. mittels Injektion, wobei die Vakzine in einer ungefähr 10^{6,0} bis ungefähr 10^{9,0} pfu enthaltenden Einzeldosisform vorliegt.

14. Verfahren zur Herstellung eines reassortanten Virus nach Anspruch 1 oder 8, umfassend die folgenden Schritte:
(a) Infektion eines geeigneten Zellsubstrats mit einer Mischinfektion bestehend aus einem Rinder-Rotavirusstamm WC3 und einem Human-Rotavirus unter Bedingungen, die eine Genreassortierung in dieser infizierten Kultur gestatten;
(b) PAGE-Untersuchung von Nachkommenschaftsklonen aus in dieser Kultur entstandenen Plaques auf das Vorhandensein entweder einer Reassortante mit zumindest dem für das v.p.4-Antigen kodierenden Genabschnitt des Rinder-Rotavirus WC3 und zwei Genen des Human-Rotavirus, wobei es sich bei einem dieser beiden Genabschnitte um den für das v.p.7-Antigen kodierenden Genabschnitt handelt, oder einer Reassortante mit zumindest dem für das v.p.4-Antigen kodierenden Genabschnitt des Human-Rotavirus und dem für das v.p.7-Antigen kodierenden Genabschnitt des Rinder-Rotavirus WC3.

15. Verfahren nach Anspruch 14, wobei das Zellsubstrat ausgewählt ist aus der aus CV-1, Vero, BSC-1, MA104 und primären Primaten-Nierenzellkulturen bestehenden Gruppe.

## Patentansprüche (Patentansprüche für folgende(n) Vertragsstaat(en): ES, GR)

1. Verfahren zur Herstellung eines reassortanten Rotavirus zur Verwendung als Bestandteil einer Vakzine gegen Infektion mit Human-Rotavirus, wobei dieses reassortante Rotavirus folgendes umfaßt:
(a) den von einem Rinder-Rotavirusstamm WC3 oder einem seiner Isolate stammenden, für das Neutralisierungsantigen v.p.4 kodierenden Genabschnitt 4, wobei dieses Isolat durch die WC3-Merkmale RNA-Elektropherotyp, Unfähigkeit zur Hämagglutination roter Blutkörperchen von Primaten, Plaque-Morphologie und Antwort im Serum-Neutralisationstest gekennzeichnet ist; sowie
(b) zumindest zwei Genabschnitte eines Human-Rotavirus, wobei es sich bei einem dieser mindestens zwei Genabschnitte um den für das Neutralisierungsantigen v.p.7 kodierenden Genabschnitt handelt;
wobei dieses reassortante Rotavirus weiterhin durch seine zurückbleibenden, ausschließlich von diesem Humanstamm, ausschließlich von diesem Rinderstamm oder von sowohl diesem Human- als auch diesem Rinderstamm stammenden Rotavirus-Genabschnitte gekennzeichnet ist und dieses reassortante Rotavirus weiterhin durch die Fähigkeit gekennzeichnet ist, in menschlichen Patienten im Erwachsenen- bzw. Kindesalter eine schützende Immunantwort auf Provokation mit nativem Rotavirus zu induzieren, ohne daß bei diesen Patienten schwerwiegende Nebenwirkungen auftreten, wobei dieses Herstellungsverfahren die folgenden Schritte umfaßt:
(a) Infektion eines geeigneten Zellsubstrats mit einer Mischinfektion bestehend aus einem Rinder-Rotavirusstamm WC3 und einem Human-Rotavirus unter Bedingungen, die eine Genreassortierung in dieser infizierten Kultur gestatten;
(b) PAGE-Untersuchung von Nachkommenschaftsklonen aus in dieser Kultur entstandenen Plaques auf das Vorhandensein einer Reassortante mit zumindest dem für das v.p.4-Antigen kodierenden Genabschnitt des Rinder-Rotavirus WC3 und zwei Genen des Human-Rotavirus, wobei es sich bei einem dieser beiden Genabschnitte um den für das v.p.7-Antigen kodierenden Genabschnitt handelt.

2. Verfahren zur Herstellung eines reassortanten Rotavirus zur Verwendung als Bestandteil einer Vakzine gegen Infektion mit reassortantem Human-Rotavirus, wobei dieses Rotavirus folgendes umfaßt:
(a) einen aus einem Rinder-Rotavirusstamm WC3 oder einem seiner Isolate stammenden Genabschnitt, wobei dieses Isolat durch die WC3-Merkmale RNA-Elektropherotyp, Unfähigkeit zur Hämagglutination roter Blutkörperchen von Primaten, Plaque-Morphologie und Antwort im Serum-Neutralisationstest gekennzeichnet ist und es sich bei diesem Genabschnitt um den für das Neutralisierungsantigen v.p.7 kodierenden Genabschnitt handelt; sowie
(b) zumindest den für das Neutralisierungsantigen v.p.4 kodierenden Genabschnitt aus einem Human-Rotavirus, wobei dieses reassortante Rotavirus weiterhin durch seine zurückbleibenden, ausschließlich von diesem Humanstamm, ausschließlich von diesem Rinderstamm oder von sowohl diesem Human- als auch diesem Rinderstamm stammenden Rotavirus-Genabschnitte gekennzeichnet ist und dieses reassortante Rotavirus weiterhin durch die Fähigkeit gekennzeichnet ist, in menschlichen Patienten im Erwachsenen- bzw. Kindesalter eine schützende Immunantwort auf Provokation mit nativem Rotavirus zu induzieren, ohne daß bei diesen Patienten schwerwiegende Nebenwirkungen auftreten, wobei dieses Herstellungsverfahren die folgenden Schritte umfaßt:
(a) Infektion eines geeigneten Zellsubstrats mit einer Mischinfektion bestehend aus einem Rinder-Rotavirusstamm WC3 und einem Human-Rotavirus unter Bedingungen, die eine Genreassortierung in dieser infizierten Kultur gestatten;
(b) PAGE-Untersuchung von Nachkommenschaftsklonen aus in dieser Kultur entstandenen Plaques auf das Vorhandensein einer Reassortante mit zumindest dem für das v.p.4-Antigen kodierenden Genabschnitt des Human-Rotavirus und dem für das v.p.7-Antigen kodierenden Genabschnitt des Rinder-Rotavirus WC3.

3. Verfahren nach Anspruch 1 oder 2, wobei das Zellsubstrat ausgewählt ist aus der aus CV-1, Vero, BSC-1, MA104 und primären Primaten-Nierenzellkulturen bestehenden Gruppe.

4. Verfahren nach einem der Ansprüche 1 bis 3, wobei dieses Human-Rotavirus aus der Gruppe bestehend aus den Viren des Serotyps 1, Serotyps 2, Serotyps 3, Serotyps 4, Serotyps M69 und Serotyps WI61 ausgewählt ist.

5. Verfahren nach Anspruch 1 oder nach dem von Anspruch 1 abhängigen Anspruch 3 oder 4, wobei es sich bei dem für das Neutralisierungsantigen v.p.7 kodierenden Human-Genabschnitt um den Genabschnitt 9 handelt.

6. Verfahren nach Anspruch 5, wobei es sich bei dieser Reassortante um WI61-7,9 handelt und diese Reassortante durch die Genabschnitte 1-6, 8 und 10-11 des Rinder-Rotavirusstamms WC3 und die Genabschnitte 7 und 9 des Humanstamms WI61 gekennzeichnet ist.

7. Verfahren nach Anspruch 1 oder nach dem von Anspruch 1 abhängigen Anspruch 3 oder 4, wobei es sich bei dem für das Neutralisierungsantigen v.p.7 kodierenden Genabschnitt um den Genabschnitt 8 handelt.

8. Verfahren nach Anspruch 7, wobei es sich bei dieser Reassortante um WI78-1, 6-11 handelt und diese Reassortante aus den Genabschnitten 2-5 des Rinder-Rotavirusstamms WC3 und den Genabschnitten 1 und 6-11 des Human-Rotavirusstamms WI78 besteht.

9. Verfahren nach Anspruch 7, wobei es sich bei dieser Reassortante um WI78-1, 7-11 handelt und diese Reassortante durch die Genabschnitte 2-6 des Rinder-Rotavirusstamms WC3 und die Genabschnitte 1 und 7-11 des Human-Rotavirusstamms WI78 gekennzeichnet ist.

10. Verfahren nach Anspruch 2 oder nach dem von Anspruch 2 abhängigen Anspruch 3 oder 4, wobei diese Reassortante aus der Gruppe bestehend aus
WI79-4, wobei diese Reassortante durch die Genabschnitte 1-3 und 5-11 des Rinder-Rotavirusstamms WC3 und den Genabschnitt 4 des Human-Rotavirusstamms WI79 gekennzeichnet ist;
WI78-4, wobei diese Reassortante durch die Genabschnitte 1-3 und 5-11 des Rinder-Rotavirusstamms WC3 und den Genabschnitt 4 des Human-Rotavirusstamms WI78 gekennzeichnet ist; und
WI61-4, wobei diese Reassortante durch die Genabschnitte 1-3 und 5-11 des Rinder-Rotavirusstamms WC3 und den Genabschnitt 4 des Human-Rotavirusstamms WI61 gekennzeichnet ist, ausgewählt ist.

## Revendications (Revendications pour l'(les) Etat(s) contractant(s) suivant(s): AT, BE, CH, DE, FR, GB, IT, LI, LU, NL, SE)

1. Réassortant de rotavirus utile à titre de composant d'un vaccin contre l'infection à rotavirus humain comprenant:
(a) le segment de gène 4, codant pour l'antigène de neutralisation v.p.4, provenant d'une souche WC3 de rotavirus bovin ou d'un isolat de celle-ci, lequel isolat est caractérisé par les caractéristiques WC3 d'électrophérotype d'ARN, d'absence d'hémagglutination avec les globules rouges de primates, de morphologie des plages, et de réponse dans le test de neutralisation par du sérum; et
(b) au moins deux segments de gène provenant d'un rotavirus humain, où l'un desdits au moins deux segments de gène est le segment de gène codant pour l'antigène de neutralisation v.p.7; ledit réassortant étant encore caractérisé par ses segments de gène de rotavirus restants ayant pour origine uniquement ladite souche humaine, uniquement ladite souche bovine ou à la fois ladite souche humaine et ladite souche bovine et ledit réassortant étant encore caractérisé comme étant susceptible d'induire une réponse immunitaire protectrice chez les malades adultes et en bas âge humains à une épreuve par le rotavirus natif sans produire d'effets néfastes graves chez lesdits malades.

2. Réassortant selon la revendication 1, caractérisé en ce que ledit rotavirus humain est choisi dans le groupe constitué du virus sérotype 1, sérotype 2, sérotype 3, sérotype 4, sérotype M69, et sérotype WI61.

3. Réassortant selon la revendication 1, caractérisé en ce que ledit segment de gène humain codant pour l'antigène de neutralisation v.p.7 est le segment de gène 9.

4. Réassortant selon la revendication 3, caractérisé en ce que ledit réassortant est WI61-7,9, ledit réassortant étant caractérisé par les segments de gène 1-6, 8 et 10-11 de la souche WC3 de rotavirus bovin et les segments de gène 7 et 9 de la souche WI61 humaine.

5. Réassortant selon la revendication 1, caractérisé en ce que ledit segment de gène humain codant pour l'antigène de neutralisation v.p.7 est le segment de gène 8.

6. Réassortant selon la revendication 5, caractérisé en ce que ledit réassortant est WI78-1,6-11, ledit réassortant étant constitué des segments de gène 2-5 de la souche WC3 de rotavirus bovin et des segments de gène 1 et 6-11 de la souche WI78 de rotavirus humain.

7. Réassortant selon la revendication 5, caractérisé en ce que ledit réassortant est WI78-1,7-11, ledit réassortant étant caractérisé par les segments de gène 2-6 de la souche WC3 de rotavirus bovin et les segments de gène 1 et 7-11 de la souche WI78 de rotavirus humain.

8. Réassortant de rotavirus utile à titre de composant d'un vaccin contre l'infection à rotavirus humain comprenant
(a) un segment de gène contribué par une souche WC3 de rotavirus bovin ou un isolat de celle-ci, lequel isolat est caractérisé par les caractéristiques WC3 d'électrophérotype d'ARN, d'absence d'hémagglutination avec les globules rouges de primates, de morphologie des plages, et de réponse dans le test de neutralisation par du sérum, ledit segment de gène étant le segment de gène codant pour l'antigène de neutralisation v.p.7; et
(b) au moins le segment de gène codant pour l'antigène de neutralisation v.p.4 provenant du rotavirus humain, ledit réassortant étant encore caractérisé par ses segments de gène de rotavirus restants ayant pour origine uniquement ladite souche humaine, uniquement ladite souche bovine ou à la fois ladite souche humaine et ladite souche bovine et ledit réassortant étant encore caractérisé comme étant susceptible d'induire une réponse immunitaire protectrice chez les malades adultes et en bas âge humains à une épreuve par le rotavirus natif sans produire d'effets néfastes graves chez lesdits malades.

9. Réassortant de rotavirus utile à titre de composant d'un vaccin contre l'infection à rotavirus humain selon la revendication 8, constitué du segment de gène codant pour l'antigène de neutralisation v.p.4 provenant d'un rotavirus humain et de dix segments de gène contribué par une souche WC3 de rotavirus bovin ou un isolat de celle-ci, lequel isolat est caractérisé par les caractéristiques WC3 d'électrophérotype d'ARN, d'absence d'hémagglutination avec les globules rouges de primates, de morphologie des plages, et de réponse dans le test de neutralisation par du sérum, ledit réassortant étant encore caractérisé comme étant susceptible d'induire une réponse immunitaire protectrice chez les malades adultes et en bas âge humains à une épreuve par le rotavirus natif sans produire d'effets néfastes graves chez lesdits malades.

10. Réassortant selon la revendication 8, caractérisé en ce que ledit rotavirus humain est choisi dans le groupe constitué du virus sérotype 1, sérotype 2, sérotype 3, sérotype 4, sérotype M69 et sérotype WI61.

11. Réassortant selon la revendication 8, choisi dans le groupe constitué de
WI79-4, ledit réassortant étant caractérisé par les segments de gène 1-3 et 5-11 de la souche WC3 de rotavirus bovin et le segment de gène 4 de la souche WI79 de rotavirus humain;
WI78-4, ledit réassortant étant caractérisé par les segments de gène 1-3 et 5-11 de la souche WC3 de rotavirus bovin et le segment de gène 4 de la souche WI78 de rotavirus humain; et
WI61-4, ledit réassortant étant caractérisé par les segments de gène 1-3 et 5-11 de la souche WC3 de rotavirus bovin et le segment de gène 4 de la souche WI61 de rotavirus humain.

12. Vaccin susceptible d'être utilisé de manière sûre chez les humains, destiné à l'immunisation contre l'infection à rotavirus humain comprenant un support pharmaceutique contenant au moins un réassortant de rotavirus choisi dans le groupe constitué
(a) d'un réassortant de rotavirus selon la revendication 1; et
(b) d'un réassortant de rotavirus selon la revendication 8.

13. Vaccin selon la revendication 12, destiné à l'administration aux êtres humains par voie orale ou nasale ou par injection, le vaccin étant sous la forme d'une dose unitaire comprenant d'environ 10^{6,0} à environ 10^{9,0} ufp.

14. Procédé de préparation d'un réassortant de virus selon la revendication 1 ou la revendication 8, comprenant les étapes consistant à:
(a) infecter un substrat cellulaire convenable par une infection mixte par une souche WC3 de rotavirus bovin et un rotavirus humain dans des conditions permettant le réassortiment de gènes dans ladite culture infectée;
(b) examiner les clones de la progéniture provenant de plages produites dans ladite culture par PAGE pour la présence soit d'un réassortant contenant au moins le segment de gène du rotavirus bovin WC3 codant pour l'antigène v.p.4 et deux gènes de rotavirus humain, où l'un desdits deux segments de gène est le segment de gène codant pour l'antigène v.p.7, soit d'un réassortant contenant au moins le segment de gène de rotavirus humain codant pour l'antigène v.p.4 et le segment de gène du rotavirus bovin WC3 codant pour l'antigène v.p.7.

15. Procédé selon la revendication 14, caractérisé en ce que ledit substrat cellulaire est choisi dans le groupe constitué de CV-1, de Vero, de BSC-1, de MA104 et de cultures primaires de cellules de rein de primates.

## Revendications (Revendications pour l'(les) Etat(s) contractant(s) suivant(s): ES, GR)

1. Procédé de préparation d'un réassortant de rotavirus utile à titre de composant d'un vaccin contre l'infection à rotavirus humain, ledit réassortant de rotavirus comprenant:
(a) le segment de gène 4, codant pour l'antigène de neutralisation v.p.4, provenant d'une souche WC3 de rotavirus bovin ou d'un isolat de celle-ci, lequel isolat est caractérisé par les caractéristiques WC3 d'électrophérotype d'ARN, d'absence d'hémagglutination avec les globules rouges de primates, de morphologie des plages, et de réponse dans le test de neutralisation par du sérum; et
(b) au moins deux segments de gène provenant d'un rotavirus humain, où l'un desdits au moins deux segments de gène est le segment de gène codant pour l'antigène de neutralisation v.p.7; ledit réassortant de rotavirus étant encore caractérisé par ses segments de gène de rotavirus restants ayant pour origine uniquement ladite souche humaine, uniquement ladite souche bovine ou à la fois ladite souche humaine et ladite souche bovine et ledit réassortant de rotavirus étant encore caractérisé comme étant susceptible d'induire une réponse immunitaire protectrice chez les malades adultes et en bas âge humains à une épreuve par le rotavirus natif sans produire d'effets néfastes graves chez lesdits malades,
ledit procédé de préparation comprenant les étapes consistant à:
(a) infecter un substrat cellulaire convenable par une infection mixte par une souche WC3 de rotavirus bovin et un rotavirus humain dans des conditions permettant le réassortiment de gènes dans ladite culture infectée;
(b) examiner les clones de la progéniture provenant de plages produites dans ladite culture par PAGE pour la présence d'un réassortant contenant au moins le segment de gène du rotavirus bovin WC3 codant pour l'antigène v.p.4 et deux gènes de rotavirus humain, où l'un desdits deux segments de gène est le segment de gène codant pour l'antigène v.p.7.

2. Procédé de préparation d'un réassortant de rotavirus utile à titre de composant d'un vaccin contre l'infection à réassortant de rotavirus humain, ledit rotavirus comprenant
(a) un segment de gène contribué par une souche WC3 de rotavirus bovin ou un isolat de celle-ci, lequel est caractérisé par les caractéristiques WC3 d'électrophérotype d'ARN, d'absence d'hémagglutination avec les globules rouges de primates, de morphologie des plages, et de réponse dans le test de neutralisation par du sérum, ledit segment de gène étant le segment de gène codant pour l'antigène de neutralisation v.p.7; et
(b) au moins le segment de gène codant pour l'antigène de neutralisation v.p.4 provenant d'un rotavirus humain, ledit réassortant de rotavirus étant encore caractérisé par ses segments de gène de rotavirus restants ayant pour origine uniquement ladite souche humaine, uniquement ladite souche bovine ou à la fois ladite souche humaine et ladite souche bovine et ledit réassortant de rotavirus étant encore caractérisé comme étant susceptible d'induire une réponse immunitaire protectrice chez les malades adultes et en bas âge humains à une épreuve par le rotavirus natif sans produire d'effets néfastes graves chez lesdits malades,
ledit procédé de préparation comprenant les étapes consistant à:
(a) infecter un substrat cellulaire convenable par une infection mixte par une souche WC3 de rotavirus bovin et un rotavirus humain dans des conditions permettant le réassortiment de gènes dans ladite culture infectée;
(b) examiner les clones de la progéniture provenant de plages produites dans ladite culture par PAGE pour la présence d'un réassortant contenant au moins le segment de gène de rotavirus humain codant pour l'antigène v.p.4 et le segment de gène du rotavirus bovin WC3 codant pour l'antigène v.p.7.

3. Procédé selon la revendication 1 ou 2, caractérisé en ce que ledit substrat cellulaire est choisi dans le groupe constitué de CV-1, de Vero, de BSC-1, de MA104 et de cultures primaires de cellules de rein de primates.

4. Procédé selon l'une quelconque des revendications 1 à 3, caractérisé en ce que ledit rotavirus humain est choisi dans le groupe constitué du virus sérotype 1, sérotype 2, sérotype 3, sérotype 4, sérotype M69, et sérotype WI61.

5. Procédé selon la revendication 1 ou selon la revendication 3 ou 4 dépendante de la revendication 1, caractérisé en ce que ledit segment de gène humain codant pour l'antigène de neutralisation v.p.7 est le segment de gène 9.

6. Procédé selon la revendication 5, caractérisé en ce que ledit réassortant est WI61-7,9, ledit réassortant étant caractérisé par les segments de gène 1-6, 8 et 10-11 de la souche WC3 de rotavirus bovin et les segments de gène 7 et 9 de la souche WI61 humaine.

7. Procédé selon la revendication 1 ou selon la revendication 3 ou 4 dépendante de la revendication 1, caractérisé en ce que ledit segment de gène humain codant pour l'antigène de neutralisation v.p.7 est le segment de gène 8.

8. Procédé selon la revendication 7, caractérisé en ce que ledit réassortant est WI78-1,6-11, ledit réassortant étant constitué des segments de gène 2-5 de la souche WC3 de rotavirus bovin et des segments de gène 1 et 6-11 de la souche WI78 de rotavirus humain.

9. Procédé selon la revendication 7, caractérisé en ce que ledit réassortant est WI78-1,7-11, ledit réassortant étant caractérisé par les segments de gène 2-6 de la souche WC3 de rotavirus bovin et les segments de gène 1 et 7-11 de la souche WI78 de rotavirus humain.

10. Procédé selon la revendication 2 ou selon la revendication 3 ou 4 dépendante de la revendication 2, caractérisé en ce que ledit réassortant est choisi dans le groupe constitué de
WI79-4, ledit réassortant étant caractérisé par les segments de gène 1-3 et 5-11 de la souche WC3 de rotavirus bovin et le segment de gène 4 de la souche WI79 de rotavirus humain;
WI78-4, ledit réassortant étant caractérisé par les segments de gène 1-3 et 5-11 de la souche WC3 de rotavirus bovin et le segment de gène 4 de la souche WI78 de rotavirus humain; et
WI61-4, ledit réassortant étant caractérisé par les segments de gène 1-3 et 5-11 de la souche WC3 de rotavirus bovin et le segment de gène 4 de la souche WI61 de rotavirus humain.
